## Europäisches Patentamt

## European Patent Office

## Office européen des brevets

(11) Veröffentlichungsnummer: **0 072 341**
**A1**

(12) **EUROPÄISCHE PATENTANMELDUNG**

(21) Anmeldenummer: 82730100.3

(22) Anmeldetag: 28.07.82

(51) Int. Cl.³: **C 08 L 75/08**, C 08 L 83/04, A 61 L 17/00

(30) Priorität: **28.07.81 DE 3130646**

(43) Veröffentlichungstag der Anmeldung: **16.02.83** Patentblatt 83/7

(84) Benannte Vertragsstaaten: **AT CH DE FR GB LI SE**

(71) Anmelder: **Lemm, Wilfried, Dr.-Ing., Mörchinger Strasse 36, D-1000 Berlin 37 (DE)**

(72) Erfinder: **Lemm, Wilfried, Dr.-Ing., Mörchinger Strasse 36, D-1000 Berlin 37 (DE)**

(74) Vertreter: **Jander, Dieter, Dipl.-Ing. et al, Dipl.-Ing. Dieter Jander Dr.-Ing. Manfred Böning Patentanwälte Kurfürstendamm 66, D-1000 Berlin 15 (DE)**

(54) **Verfahren zum Herstellen von Gegenständen aus Polyurethan, die in gewissen Oberflächenbereichen mit Polydimethylsiloxan angereichert sind.**

(57) Verfahren zur Herstellung von Gegenständen aus Polyurethan, die in gewissen Oberflächenbereichen mit Polydimethylsiloxan angereichert sind, bei welchem Verfahren eine erste Lösung aus Polyurethan in wasserfreiem Dimethylacetamid und eine zweite Lösung aus Polydimethylsiloxan, das bei Zimmertemperatur und in Gegenwart von Luftfeuchtigkeit vulkanisiert, in wasserfreiem Tetrahydrofuren miteinander vermischt werden und die Mischung, in die Form des Gegenstandes gebracht, trocknet. Die Erfindung besteht darin, dass die erste Lösung, die ein Polyätherurethan-Elastomer als Polyurethan enthält, 2 bis 7%ig, insbesondere 5%ig, ist, dass man das Polyätherurethan-Elastomer in dem Dimethylacetamid derart schonend löst, dass die Molekülstruktur des Polyätherurethan-Elastomers weitgehend erhalten bleibt, dass die Lösung anschliessend kurz derart erhitzt wird, dass man eine klare homogene Lösung erhält, dass man diese derart erkalten lässt, dass sich ein filtrierbarer Niederschlag bildet, dass dieser abfiltriert wird, dass die zweite Lösung 0,5-4%ig, insbesondere 0,8-3%ig, insbesondere 1%ig, ist und dass die Mischung bei Temperaturen zwischen 50 und 60°C, insbesondere bei 60°C. getrocknet wird.

## Verfahren zum Herstellen von Gegenständen aus Polyurethan, die in gewissen Oberflächenbereichen mit Polydimethylsiloxan angereichert sind.

Die Erfindung bezieht sich auf ein Verfahren zum Herstellen von Gegenständen aus Polyurethan, die in gewissen Oberflächenbereichen mit Polydimethylsiloxan angereichert sind, bei welchem Verfahren eine erste Lösung aus Polyurethan in wasserfreiem Dimethylacetamid und eine zweite Lösung aus Polydimethylsiloxan, das bei Zimmertemperatur und in Gegenwart von Luftfeuchtigkeit vulkanisiert, in wasserfreiem Tetrahydrofuran miteinander vermischt werden und die Mischung, in die Form des Gegenstandes gebracht, trocknet.

Gegenstände dieser Art finden in der Medizin in Form von Blutgefäßprothesen, Kathetern, Herzklappen usw. Anwendung. Polydimethylsiloxan zeichnet sich in Kombination mit dem Polyurethan durch eine außerordentlich gute Blutverträglichkeit aus. Polyurethan hat zudem hervorragende mechanische Eigenschaften. Die Gegenstände kommen derart zur Anwendung, daß die mit Polydimethylsiloxan angereicherte und luftseitig getrocknete Schicht dem Blut zugewandt ist.

Das eingangs erwähnte Verfahren ist bekannt geworden aus: First World Biomaterials Congress, Vienna, Book of Abstract, 1980, 4.5.6.

Der Erfindung liegt die Aufgabe zugrunde, Bedingungen für die einwandfreie Durchführung des Verfahrens zu schaffen.

Diese Aufgabe wird erfindungsgemäß dadurch gelöst, daß die erste Lösung, die ein Polyätherurethan-Elastomer als Polyurethan enthält, 2 - 7%ig, insbesondere 5%ig, ist, daß man das Polyätherurethan-Elastomer in dem Dimethylacetamid derart schonend löst, daß die Molekülstruktur des Polyätherurethan-Elastomers weitgehend erhalten bleibt, daß die Lösung anschliessend kurz derart erhitzt wird, daß man eine klare homogene Lösung erhält, daß man diese derart erkalten läßt, daß sich ein filtrierbarer Niederschlag bildet, daß dieser abfiltriert wird, daß die zweite Lösung 0,5 - 4%ig, insbesondere 0,8 - 3%ig, insbesondere 1%ig, ist und daß die Mischung bei Temperaturen zwischen 50 und 60°C, insbesondere bei 60°C, getrocknet wird.

Es hat sich gezeigt, daß bei Einhalten dieser Bedingungen einwandfreie Ergebnisse erzielt werden:

Polyätherurethan besitzt die notwendige medizinische Reinheit und die erforderliche Basisfestigkeit.

Würde man durch Erhitzen eine schnellere Lösung des Polyätherurethans in dem Dimethylacetamid bewirken, so würde das zu einer Veränderung der Molekülstruktur des Polyätherurethans und damit zu Abbauprodukten führen, die physiologisch bedenklich sind. Auch würden dadurch die mechanischen Eigenschaften verschlechtert werden.

Durch die anschliessende kurze Erhitzung ist es möglich, die dispers verteilten Partikel des Polyätherurethans in Lösung zu bringen derart, daß eine homogene Lösung entsteht. Beim anschliessenden vorsichtigen Erkalten bildet sich ein filtrierbarer Niederschlag, der ohne weiteres abfiltriert werden kann.

-3-

Nur bei den angegebenen Konzentrationsverhältnissen ist die Mischung der sonst inkompatiblen Polymere stabil, und nur bei Einhaltung der vorgegebenen Trocknungsbedingungen ist die einwandfreie Blutverträglichkeit gegeben. Die Verhältnisse (Mischungsverhältnis, Trockungstemperatur) sind derart aufeinander abgestimmt, daß die Entmischungsgeschwindigkeit der beiden Komponenten einerseits und die Vernetzungsgeschwindigkeit der Silikonkomponente andererseits zu dem gewünschten Resultat führen.

Eine Weiterentwicklung der Erfindung besteht darin, daß man die Mischung nach dem Vergießen wenigstens 16 Stunden, insbesondere 24 Stunden, trocknet.

Zur Herstellung einer Schicht aus dem erwähnten Material auf einem Polyurethanträger wird vorgeschlagen, daß der Gehalt von Polydimethylsiloxan in Polyätherurethan 5% in der Mischung beträgt.

Zur Herstellung von Gegenständen ohne Polyurethanträger, wie Folien, Schläuchen usw. wird vorgeschlagen, daß der Gehalt von Polydimethylsiloxan in Polyätherurethan 3% in der Mischung beträgt.

Zur Beschichtung von porigen Blutgefäßprothesen sollten 2,5% Polyätherurethan in Dimethylacetamid und 0,5% Polydimethylsiloxan in Tetrahydrofuran gelöst werden. Das ergibt eine sehr verdünnte Lösung eines Gemisches von Polyätherurethan und 3% Polydimethylsiloxan, so daß beim Beschichten die Porigkeit bestehen bleibt. Zur Beschichtung wird die Gefäßprothese langsam in die verdünnte Polyätherurethan-Polydimethylsiloxan-Lösung gelegt, diese kurz evakuiert und anschließend belüftet. Die Prothese wird entnommen und bei 55-60°C in einem Trockenschrank 16 - 20 Stunden getrocknet.

- 4 -

Zweckmässig ist es, wenn man das Polyätherurethan in Dimethylacetamid bei etwa Zimmertemperatur in einem Zeitraum von etwa 5 - 7 Tagen löst.

Weiterhin ist es zweckmässig, wenn anschliessend die Lösung des Polyätherurethans kurz auf 60-70°C erhitzt wird.

Zur Beschleunigung der Herstellung der Polyätherurethan-Lösung wird vorgeschlagen, daß man das Polyätherurethan zunächst in Dimethylformamid löst, insbesondere auf 60-70°C erhitzt, bis alles in Lösung gegangen ist, nach dem Erkalten filtriert, die so entstandene homogene Lösung in einen Überschuß an Methanol insbesondere bei starkem Rühren eingibt, den dabei entstehenden amorphen Niederschlag filtriert, insbesondere bei 80°C trocknet und das auf diese Weise entstandene amorphe Polyätherurethan in Dimethylacetamid löst. Das Lösen des Polyätherurethans in dem Dimethylformamid nimmt nur etwa 3 - 4 Tage, das Lösen des Polyätherurethans in dem Dimethylacetamid nur etwa 20 - 24 Stunden in Anspruch. Außerdem fällt das anschliessende Erhitzen und Abfiltrieren fort. Das Methanol-Dimethylformamid-Gemisch kann durch fraktionierte Destillation aufbereitet werden.

Schließlich wird vorgeschlagen, daß das Polyätherurethan aus einem aromatischen Diisocyanat, einem Polyätherdiol und einem kurzkettigen Diol oder Diamin besteht.

<u>Beispiele</u>

<u>Herstellung der ersten Lösung</u>
In 950 g absolut wasserfreiem Dimethylacetamid werden bei Zimmertemperatur 50 g Polyätherurethan, herge-

- 5 -

stellt aus 4,4'-Diisocyanatodephenylmethan, Polytetramethylenglycol, 1.4-Butandiol
(= "Pellethane 2363-80A"), in Granulatform gelöst.
Der Lösungsvorgang dauert 5 - 7 Tage. Danach wird
die Lösung kurz auf 60 - 70°C erhitzt, wobei die
Lösung vollkommen klar wird. Beim Erkalten bildet
sich ein kristalliner Niederschlag, der sich durch
Vakuumfiltration entfernen läßt. Die Viskosität
dieser Lösung beträgt bei 25°C ca. 600 cps (m x Pa x s).

Herstellung der zweiten Lösung

In 990 g absolut wasserfreiem Tetrahydrofuran werden
bei Zimmertemperatur 10 g Polydimethylsiloxan ("Dow
Corning 3140 RTV") gelöst und anschliessend bei Normaldruck rasch filtriert.

Verarbeitung zu Folien bzw. Beschichtungen:

Folien mit einem Gehalt von 3% Polydimethylsiloxan,
bezogen auf die Polyurethankomponente, verfügten über
die beste Blutverträglichkeit. Verwendet man dagegen
die Polyurethan-Polydimethylsiloxan-Mischungen zur Beschichtung eines Polyurethan-Basismaterials, so hat
sich eine Lösung mit 5% Polydimethylsiloxan, bezogen
auf die Polyurethankomponente, als optimal erwiesen.

Um eine 3%ige (5%ige) Lösung herzustellen, mischt man
unter Rühren 100 g der ersten Lösung und 15 g (25 g)
der zweiten Lösung.

Die Lösungen sind bei Lagerung unter Licht- und Feuchtigkeitsausschluß mehrere Monate gebrauchsfähig.

Zur Herstellung von Gießfolien (Membranen) wird die
3%ige Lösung auf eine saubere und glatte Glasplatte

- 6 -

gegossen und im Trockenschrank bei 60°C 24 Stunden getrocknet, wobei Luftfeuchtigkeit Zutritt haben soll. Es ist zu beachten, daß nur die der Luft zugewandte Oberfläche eine hohe Blutverträglichkeit besitzt.

Zur Beschichtung von Polyurethanbasismaterial (z.B. für Rollerpumpenschläuche, Blutpumpen) verwendet man die 5%ige Gießlösung. Die Schläuche bzw. die Blutpumpen werden mit Beschichtungslösung gefüllt, die nach ca. 2 - 3 Minuten wieder ausgegossen wird. Dann trocknet man 24 Stunden bei 60°C rotierend, damit die Beschichtung gleichförmig wird. Danach wird der gesamte Vorgang ein 2. Mal wiederholt.

Die Prozent-Angaben sind stets Gewichts-Prozent-Angaben.

- 7 -

Patentansprüche :

1. Verfahren zum Herstellen von Gegenständen aus Polyurethan, die in gewissen Oberflächenbereichen mit Polydimethylsiloxan angereichert sind, bei welchem Verfahren eine erste Lösung aus Polyurethan in wasserfreiem Dimethylacetamid und eine zweite Lösung aus Polydimethylsiloxan, das bei Zimmertemperatur und in Gegenwart von Luftfeuchtigkeit vulkanisiert, in wasserfreiem Tetrahydrofuran miteinander vermischt werden und die Mischung, in die Form des Gegenstandes gebracht, trocknet,
dadurch gekennzeichnet,
daß die erste Lösung, die ein Polyätherurethan-Elastomer als Polyurethan enthält, 2 - 7%ig, insbesondere 5%ig, ist, daß man das Polyätherurethan-Elastomer in dem Dimethylacetamid derart schonend löst, daß die Molekülstruktur des Polyätherurethan-Elastomers weitgehend erhalten bleibt, daß die Lösung anschliessend kurz derart erhitzt wird, daß man eine klare homogene Lösung erhält, daß man diese derart erkalten läßt, daß sich ein filtrierbarer Niederschlag bildet, daß dieser abfiltriert wird, daß die zweite Lösung 0,5 - 4%ig, insbesondere 0,8 - 3%ig, insbesondere 1%ig, ist und daß die Mischung bei Temperaturen zwischen 50 und 60°C, insbesondere bei 60°C, getrocknet wird.

2. Verfahren nach Anspruch 1,
dadurch gekennzeichnet,
daß man die Mischung nach dem Vergießen wenigstens 16 Stunden, insbesondere 24 Stunden, trocknet.

3. Verfahren nach Anspruch 1 oder 2, dadurch gekennzeichnet, daß zur Herstellung einer Schicht aus dem erwähnten Material auf einem Polyurethan-Träger der Gehalt von Polydimethylsiloxan in Polyurethan 5% in der Mischung beträgt.

4. Verfahren nach Anspruch 1 oder 2, dadurch gekennzeichnet, daß zur Herstellung von Gegenständen ohne Polyurethan-träger, wie Folien, Schläuchen, der Gehalt von Polydimethylsiloxan in Polyurethan 3% in der Mischung beträgt.

5. Verfahren nach Anspruch 1 oder 2, dadurch gekennzeichnet, daß zur Beschichtung von porigen Blutgefäßprothesen 2,5% Polyätherurethan in Dimethylacetamid und 0,5% Polydimethylsiloxan in Tetrahydrofuran gelöst werden.

6. Verfahren nach einem oder mehreren der Ansprüche 1-5, dadurch gekennzeichnet, daß man das Polyätherurethan in Dimethylacetamid bei etwa Zimmertemperatur in einem Zeitraum von etwa 5 - 7 Tagen löst.

7. Verfahren nach einem oder mehreren der Ansprüche 1-6, dadurch gekennzeichnet, daß die Lösung des Polyurethans kurz auf 60 - 70°C erhitzt wird.

8. Verfahren nach einem oder mehreren der Ansprüche 1-5, dadurch gekennzeichnet, daß man zur Beschleunigung der Herstellung der Polyätherurethan-Lösung das Polyätherurethan zunächst in Dimethylformamid löst, insbesondere auf 60-70°C erhitzt, bis alles in Lösung gegangen ist, nach dem Erkalten filtriert, die so entstandene homogene Lösung in einen Überschuß an Methanol insbesondere bei starkem Rühren eingibt, den dabei entstehenden amorphen Niederschlag filtriert, insbesondere bei 80°C trocknet und das auf diese Weise entstandene amorphe Polyätherurethan in Dimethylacetamid löst.

9. Verfahren nach einem oder mehreren der Ansprüche 1-8, dadurch gekennzeichnet, daß das Polyätherurethan aus einem aromatischen Diisocyanat, einem Polyätherdiol und einem kurzkettigen Diol oder Diamin besteht.

Europäisches
Patentamt

**EUROPÄISCHER RECHERCHENBERICHT**

Nummer der Anmeldung

EP 82 73 0100.3

| | EINSCHLÄGIGE DOKUMENTE | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| D,P,X | "Biomaterials 1980" 1982, J. WILEY & SONS, Chichester W. LEMM et al. "Polyurethane - Polydimethylsiloxane Mixtures with Improved Thromboresistance, Their Chemical and Biological Properties" Seiten 459 bis 464 * Seite 459, Zeile 1 bis Seite 461, Zeile 7 * | 1-4 | C 08 L 75/08 C 08 L 83/04 A 61 L 17/00 |
| | | | RECHERCHIERTE SACHGEBIETE (Int. Cl.³) |
| P,A | GB - A - 2 073 219 (THORATEC LABORATORIES CORP.) * Seite 3, Zeilen 103 bis 109 * & DE - A - 3 107 542 | 1 | A 61 L 17/00 C 08 L 75/00 C 08 L 83/04 |
| A | GB - A - 1 253 760 (AVCO CORP.) * Seite 2, Zeilen 54 bis 111 * & DE - A - 1 944 969 | 9 | |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Berlin | 19-10-1982 | IDEZ |

EPA form 1503.1 06.78